# EUROPÄISCHE PATENTSCHRIFT

(11) **EP 0 715 613 B1**
(45) Veröffentlichungstag und Bekanntmachung des Hinweises auf die Patenterteilung: **28.01.1998**
(21) Anmeldenummer: 94926896.5
(22) Anmeldetag: 26.08.1994
(51) Int. Cl.: C07C 15/52, A61K 31/015

(54) **NEUES DIPHENYLHEXEN**
NEW DIPHENYLHEXENE
NOUVEAU DIPHENYLHEXENE

(30) Priorität: 27.08.1993 DE 4328965
(43) Veröffentlichungstag der Anmeldung: 12.06.1996
(73) Patentinhaber: PECHAN, Reinhard, 81925 München (DE); METZLER, Manfred, D-76229 Karlsruhe (DE)
(72) Erfinder: PECHAN, Reinhard, 81925 München (DE); METZLER, Manfred, D-76229 Karlsruhe (DE)
(74) Vertreter: Huber, Bernhard, Dipl.-Chem.
(86) Internationale Anmeldenummer: EP9402837
(87) Internationale Veröffentlichungsnummer: WO9506020

(56) Entgegenhaltungen:
- WO-A-93/22262
- CHEMISCHE BERICHTE, Bd.115, Nr.12, 1982, WEINHEIM DE Seiten 3697 - 3705 J. LEIMNER ET AL 'Stereochemie und Nebenprodukte der reduktiven Kupplung von Alkylarylketonen zu 1,2-Dialkyl-1,2-diarylethylenen'

## Beschreibung

Die vorliegende Erfindung betrifft die chemische Verbindung Diphenyl-3-hexen, ein Verfahren zu ihrer Herstellung und ihre Anwendung als Therapeutika.

Einer Vielzahl von Erkrankungen des Menschen liegt ein unkontrolliertes Wachstum von Körperzellen zugrunde. Diese Proliferationsstörung kann zu einer benignen Entartung, z.B. Psoriasis vulgaris bei Keratinozyten oder β-Thalassämie bei Erythrozyten oder aber auch zu einer malignen Entartung, z.B. bei allen Formen von Krebs, führen.

Das Ziel einer Krebstherapie ist die vollständige Zerstörung, zumindest aber eine signifikante Wachstumshemmung der Tumorzellen. Neben chirurgischen Maßnahmen und Strahlentherapie stehen heute auch Chemotherapeutika, z.B. 5-Fluoruracil, Cytosin-Arabinosid etc., zur Behandlung von Krebspatienten zur Verfügung. Ein Nachteil bei der Verwendung dieser Chemotherapeutika besteht jedoch darin, daß sie neben den Tumorzellen auch gesunde Bereiche des Organismus schädigen können. Daher wurde seit mehreren Jahren nach weniger aggressiven Behandlungsmethoden gesucht. So erbrachte die Anwendung von Tamoxifen bei Mammakarzinomen erste vielversprechende Resultate (Jordan, Antiestrogens in Cancer Treatment, in: Stoll, B.A., Endocrine Management of Cancer, Karger, Basel (1988), 57-65). Bis heute ist jedoch eine Vielzahl von Krebserkrankungen (z.B. Kolon- und Pankreaskarzinom) noch nicht in ausreichendem Umfang durch Medikamente therapierbar (Cohen et al., Colorectal Cancer, und Brennan et al., Cancer of the Pancreas in: DeVita, V.T., Helman, S. und Rosenberg, St. A., Cancer, Lippincott Co., 3rd Edition (1989)).

Als eine der Hauptursachen der Krebsentstehung gelten Fehler bei der Expression von sogenannten Onkogenen, wie etwa myc oder ras (Tabin et al., Nature, 300 (1982), 143-149). Ein wesentlicher Regulationsmechanismus der Genexpression - auch bei Onkogenen - ist der Methylierungsgrad der DNA (Doerfler, J. gen. Virol., 57 (1981) 1-20). So konnte am Beispiel der ras- und myc-Onkogene eine Hypomethylierung im exprimierten, kanzerogenen Status nachgewiesen werden (Feinberg et al., Biophys. Res. Comm., 111 (1983) 47-54; Cheah et al., J. Nat. Cancer Inst., 73, (1984), 1057-1061)). Weiterhin ist bekannt, daß durch eine gezielte Steuerung der DNA-Methylierung sowohl das Wachstum als auch die korrekte somatische Funktion von entarteten Zellen reguliert werden kann. Dies wurde von Ley et al. (DNA-methylation and globin gene expression in patients treated with 4-azacytidine, in: Globin Gene Expression and Hematopoietic Differentiation, Alan P. Liss, N.Y. (1983), 457-474) gezeigt. Ein weiterer direkter Zusammenhang zwischen der Methylierung von DNA und Störungen im zentralen Nervensystem wurde bei HIV-positiven Patienten gefunden. (Keating et al., Lancet, 337 (1991) 935-939).

Ferner konnte bei in vitro Zellkulturversuchen ein direkter Zusammenhang zwischen maligner Transformation und DNA-Hypomethylierung einerseits sowie zwischen konzentrationsabhängiger Wachstumsinhibierung von Tumorzellen (aus Mensch und Tier) und DNA-Hypermethylierung andererseits nachgewiesen werden (Pechan, (1987), Dissertation, Universität Würzburg).

Eine Aufgabe der vorliegenden Erfindung war die Bereitstellung einer Verbindung, die eine pharmazeutische Wirksamkeit bei der Behandlung von Erkrankungen zeigten, die im Zusammenhang mit entartetem Zellwachstum, d.h. Störungen der Zellproliferation, stehen.

Die erfindungsgemäße Aufgabe wird durch 3,4-Diphenyl-3-hexen gelöst.

Die Verbindung ist durch ein Verfahren erhältlich, welches dadurch gekennzeichnet ist, daß man 1-Phenyl-1-propanon in Gegenwart eines Übergangsmetallchlorids, Zinkstaub und einer Base in einem inerten Lösungsmittel umsetzt und die gewünschte Verbindung aus dem Reaktionsgemisch durch Aufreinigung gewinnt.

Das Übergangsmetallchlorid ist vorzugsweise Titantetrachlorid und die Base ist vorzugsweise Pyridin. Das inerte Lösungsmittel ist vorzugsweise 1,4-Dioxan und die Umsetzung erfolgt günstigerweise bei der Rückflußtemperatur des Lösungsmittels. Weitere Einzelheiten dieses Verfahrens sind in einer Arbeit von McMurry (J. Org. Chem. 54 (1989), 3748-3749) beschrieben.

Überraschenderweise zeigten die erfindungsgemäße Substanz eine signifikante Wachstumsinhibition von Tumorzellen in vitro. Daher ist zu erwarten, daß sich die Verbindung als Therapeutikum zur Behandlung von Erkrankungen eignet, die im Zusammenhang mit entartetem Zellwachstum stehen.

Ein weiterer Gegenstand der vorliegenden Erfindung ist daher eine pharmazeutische Zusammensetzung, welche die erfindungsgemäße Verbindung als Wirkstoff und gegebenenfalls pharmazeutisch übliche Träger-, Füll-, Verdünnungs- oder/und Hilfsstoffe enthält.

Die Verbindung eignet sich für alle Indikationsgebiete der Human- und Veterinärmedizin, bei denen eine Wachstumshemmung von entarteten Körperzellen oder/und deren Beeinflussung aufgrund einer DNA-Hypermethylierung erfolgen kann. Insbesondere sind hier Krebs, Psoriasis, oder AIDS zu nennen.

Die erfindungsgemäße pharmazeutische Zusammensetzung kann dabei in beliebigen Formulierungen verabreicht werden, welche die erfindungsgemäße Verbindunge enthalten. Beispiele für geeignete Formulierungen sind etwa Tabletten, Kapseln, Granulate, Lösungen, Salben oder Aerosole. Die Herstellung derartiger Formulierungen ist einem Fachmann auf dem Gebiet der Pharmazie hinreichend bekannt, so daß eine ausführliche Beschreibung nicht erforderlich scheint.

Das folgende Beispiel dient zur weiteren Verdeutlichung der Erfindung.

### Beispiel 1

### Synthese von 3,4-Diphenyl-3-hexenen nach McMurry (1989, supra)

Zu 200 ml 1,4-Dioxan werden unter Rühren (Eiskühlung, Argonatmosphäre).108 mmol Titantetrachlorid zugetropft. Dieser Lösung werden in kleinen Portionen 200 mmol Zinkstaub und 8 ml Pyridin zugegeben. Nach langsamer (30 min) Zugabe von 1-(4-Alkylphenyl)-1-propanon wird die Reaktionslösung unter Rückfluß erhitzt (20 h). Nach dem Erkalten wird diese Lösung mit 200 ml 10%iger Kaliumcarbonatlösung versetzt. Die so entstandene Suspension wird mit 50 ml Diethylether ausgeschüttelt, mit Wasser gewaschen und über Magnesiumsulfat getrocknet.

Zur Reinigung wird das Rohprodukt über Kieselgel mit Petrolether/Diethylether (4/1) filtriert und anschließend durch Säulenchromatographie (Kieselgel, Petrolether/Diethylether 2/1) getrennt. Die Reinsubstanz (farblos) kristallisiert in der Kälte nach mehreren Tagen.
- 3,4-Diphenyl-3-hexen: Ausbeute: 27 %
Schmelzpunkt: 72,2°C

### Beispiel 2

### Wachstumsinhibition von Tumorzellen in vitro

In Zellkulturflaschen (T25, Greiner) wurden 10⁴ bis 10⁵ Tumorzellen in jeweils 3 ml IBR-Medium (15% Fetales Kälberserum, 25 U/ml Penicillin-Streptomycin) ausgesät. Nach einer Anwachsphase von 4h (37°C, 12% CO₂) wurden 50 µM Testsubstanz in 0.1% DMSO gelöst, sowie 0,1% DMSO (Kontrolle), zugegeben. Als Testsubstanz wurde die Verbindung von Beispiel 1 verwendet.

Die Zählung der Zellen (Neubauer-Zählkammer) erfolgte in Abständen von 24 h, über einen Zeitraum von 9 Tagen. Nach 3 und 6 Tagen wurde das Medium gewechselt und die Testsubstanz (siehe Beispiel 1) erneut in gleicher Konzentration zugegeben.

Das Wachstum von HT29 Leberkarzinomzellen (Mensch) konnte konzentrationsabhängig bis zu 90 % inhibiert werden, das von in vitro transformierten Hamsterzellen um etwa 30%.

## Patentansprüche

1. Pharmazeutische Zusammensetzung,
**dadurch gekennzeichnet,**
daß sie 3,4-Diphenyl-3-hexen als Wirkstoff und ggf. pharmazeutisch übliche Träger-, Füll-, Verdünnungs- oder/und Hilfsstoffe enthält.

2. Verwendung von 3,4-Diphenyl-3-hexen zur Herstellung einer pharmazeutischen Zusammensetzung zur Behandlung von Erkrankungen, die im Zusammenhang mit entartetem Zellwachstum stehen.

3. Verwendung nach Anspruch 2 zur Behandlung von Krebs, Psoriasis oder AIDS.

## Claims

1. Pharmaceutical composition,
**wherein**
it contains 3,4-diphenyl-3-hexene as the active substance and if desired common pharmaceutical carriers, fillers, diluents or/and auxiliary substances.

2. Use of 3,4-diphenyl-3-hexene for the treatment of diseases that are associated with degenerate cell growth.

3. Use as claimed in claim 2 for the treatment of cancer, psoriasis or AIDS.

## Revendications

1. Composition pharmaceutique, caractérisée en ce qu'elle contient du 3,4-diphényl-3-hexène à titre de principe actif et, éventuellement des excipients, charges, diluants ou/et adjuvants courants en pharmacie.

2. Utilisation de 3,4-diphényl-3-hexène pour la préparation d'une composition pharmaceutique pour le traitement de maladies qui sont en rapport avec une croissance cellulaire dégénérée.

3. Utilisation selon la revendication 2 pour le traitement du cancer, du psoriasis ou du Sida.
